(19) Europäisches Patentamt
European Patent Office
Office européen des brevets

(11) Numéro de publication : **0 105 777**
**B1**

# (12) FASCICULE DE BREVET EUROPÉEN

(45) Date de publication du fascicule du brevet : **16.04.86**

(21) Numéro de dépôt : **83401809.5**

(22) Date de dépôt : **15.09.83**

(51) Int. Cl.[4] : **C 07 K 5/00**, C 07 K 7/00, C 07 K 1/00, C 08 G 69/08, C 09 K 19/02

(54) **Lipopeptides, leur obtention et leur application comme émulsifiants.**

(30) Priorité : **22.09.82 FR 8215976**

(43) Date de publication de la demande : **18.04.84 Bulletin 84/16**

(45) Mention de la délivrance du brevet : **16.04.86 Bulletin 86/16**

(84) Etats contractants désignés : **BE CH DE FR GB IT LI LU NL**

(56) Documents cités :
**CHEMICAL ABSTRACTS, vol. 97, no. 23, 1982, page 2, no. 192611x, Columbus, Ohio, USA G.H. WERNER et al.: "Low molecular weight synthetic lipopeptides: a new class of immunopotentiating substances"**
**CHEMICAL ABSTRACTS, vol. 73, 1970, page 4, no. 116276f, Columbus, Ohio, USA N.K. GARG et al.: "Amino acid containing lipids: lipoamine acids, peptidolipids, and proteolipids"**
**MOL. CRYST. LIQ. CRYST., vol. 63, 1981, pages 205-214, Gordon and Breach Science Publishers, Inc., USA K. CZARNIECKA et al.: "Polypeptide liquid crystals: A deuterium NMR study"**

(73) Titulaire : **Etablissement Public dit: CENTRE NATIONAL DE LA RECHERCHE SCIENTIFIQUE (CNRS)**
**15, Quai Anatole France**
**F-75700 Paris (FR)**

(72) Inventeur : **Gallot, Bernard René Maurice**
**220 Rue Rodolphe Richard**
**F-45160 Olivet (FR)**
Inventeur : **Douy, André**
**269 Rue des Briandes**
**F-45160 Olivet (FR)**

(74) Mandataire : **Phélip, Bruno et al**
**c/o Cabinet Harlé & Phélip 21, rue de la Rochefoucauld**
**F-75009 Paris (FR)**

**EP 0 105 777 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne la synthèse de lipopeptides, et plus particulièrement de lipopeptides amphipatiques, et l'application de ces composés comme émulsifiants ou comme cristaux liquides.

L'invention a pour premier objet une nouvelle classe de lipopeptides, qui sont des lipopeptiques amphipatiques composés d'une chaîne hydrophobe comprenant au moins 8 atomes de carbone environ, et de préférence de 8 à 24 atomes de carbone environ, et d'une chaîne peptidique hydrophile ou rendue hydrophile.

Des lipopeptides, non amphipatiques ont été précédemment décrites. Ainsi dans Dev. Immunol. 534-43 (1982), est rapporté une étude de la stimulation des réactions immunitaires par des lipopeptides qui résultent de la réaction d'un acide gras sur la fonction amine en alpha d'un tri-, tétra- ou pentapeptide. Par ailleurs, dans J. Sci. Ind. Res. 197-202 (1970), on décrit l'extraction à partir de milieux naturels, animaux, plantes ou bactéries de divers lipopeptides et lipoaminoacides, dont certains ont des activités biologiques qui pourraient résulter de leur intervention dans les mécanismes de la biosynthèse protéique ; il est indiqué que les lipopeptides, dont la structure a été déterminée, sont des dérivés d'acides gras fixés sur diverses polypeptides, plus ou moins estérifiés, par une liaison amide.

Par contre dans les lipopeptides selon l'invention, la partie lipidique dérive d'une amine grasse, qui est engagée dans une fonction amide avec la fonction carboxylique du polypeptide.

Les lipopeptides selon l'invention peuvent être définis par la formule générale :

CnPP

dans laquelle Cn représente une chaîne hydrophobe ayant au moins 8 atomes de carbone environ, et de préférence 8 à 24 atomes de carbone environ, n désignant le nombre d'atomes de carbone, et PP représente un polypeptide obtenu à partir des acides aminés naturels ou de leurs dérivés (de configuration l ou d). En pratique, le polypeptide PP est formé d'un ou plusieurs acides aminés, selon le degré de polymérisation retenu, ce dernier pouvant être de 1 ou plus. La nomenclature de certaines, parmi les plus courantes, des séquences peptidiques qu'il est possible d'utiliser est regroupée plus loin dans le tableau I.

Par chaîne hydrophobe, on entend de préférence, mais non exclusivement, une chaîne hydrocarbonée aliphatique, éventuellement substituée, ayant un nombre d'atomes de carbone tel qu'indiqué.

On a obtenu ces composés selon une technique elle-même nouvelle.

L'invention a donc également pour objet un procédé pour l'obtention de lipopeptides amphipatiques tels que définis ci-dessus, consistant fondamentalement à :

1. réaliser un couplage peptidique entre une amine grasse et un amino acide N-protégé, pour obtenir un lipopeptide dont la chaîne peptidique a un degré de polymérisation de 1, et, si l'on désire obtenir un degré de polymérisation de 2 ou 3 pour la chaîne peptidique, réaliser un autre couplage peptidique d'un amino acide N-protégé sur le produit de degré de polymérisation immédiatement inférieur, ou

2a. effectuer la polymérisation du N-carboxyanhydride de l'amino acide en l'initiant par l'amine grasse $CnNH_2$, pour obtenir des lipopeptides dont les chaînes peptidiques ont un degré de polymérisation qui dépendra des conditions opératoires choisies, et

2b. si on le désire, fractionner en composition les lipopeptides de l'étape 2a, et

3. excepté dans le cas où la chaîne peptidique du produit de l'étape 1 et/ou 2a ou 2b est directement une chaîne hydrophile, débloquer les chaînes latérales de la chaîne peptidique hydrophobe pour les rendre hydrophiles.

Pour obtenir des lipopeptides à degré de polymérisation 1, 2 ou 3, on procède par exemple par couplage peptidique entre l'amine grasse de formule $CnNH_2$, où Cn est tel que précédemment défini, et l'amino acide dont l'atome d'azote amino est protégé, par exemple par le groupement tert-butyloxycarbonyle (en abrégé Boc) et on débloque cet atome d'azote amino pour obtenir le produit de degré de polymérisation 1, sur lequel on refera un couplage avec l'amino acide bloqué pour obtenir après débloquage de l'azote terminal le produit de degré de polymérisation 2 et ainsi de suite.

Pour obtenir les lipopeptides selon l'invention, on peut aussi polymériser le N-carboxy-anhydride (en abrégé NCA) de l'amino acide en initiant la polymérisation par l'amine grasse $CnNH_2$. On réalise ensuite, si on le désire, un fractionnement en composition (la séquence lipidique étant monodispersée), par précipitation sélective des lipopeptides, et on obtient une série de lipopeptides qui diffèrent par leur composition en peptides.

Les peptides monomères mis en œuvre sont des produits commerciaux ou sont préparés de manière connue.

Le monomère utilisé pour la partie peptidique des lipopeptides selon l'invention n'est donc pas l'acide aminé lui-même, ce sera son dérivé cyclique, le N-carboxy-anhydride d'amino-acide (NCA), obtenu par action du phosgène sur l'amino acide selon la réaction :

(Voir dessin page 3)

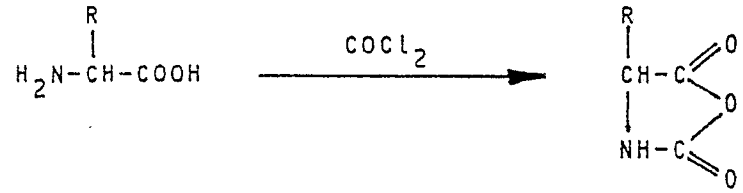


ou l'acide aminé N protégé par un des groupes habituellement utilisés en synthèse peptidique.

On prépare les NCA dans le THF, en faisant réagir sur l'amino acide une solution de phosgène dans le tétrahydrofuranne (THF). Cette méthode est une variante de la méthode de Fuller, Verlander et Goodman (Biopolymers, *15* (1869/1976), dans laquelle le solvant du phosgène est le benzène.

L'amine grasse $CnNH_2$ est soit une amine commercialement disponible, soit obtenue à partir de l'acide gras ayant un atome de carbone de plus par la réaction de dégradation de Schmidt utilisant l'azide de sodium en milieu acide fort [Indian J. Technol., *5*, 262 (1967)], soit obtenue par couplage entre le chlorure d'acide acrylique ou méthacrylique et une diamine primaire, ou un amino alcool N protégé. Le choix d'une chaîne Cn ayant de 8 à 24 atomes de carbone environ n'est pas critique, mais tient uniquement à la plus grande disponibilité des produits correspondants. Cette chaîne hydrophobe Cn peut être une chaîne hydrocarbure quelconque, mais elle peut aussi comporter des substituants et/ou des hétéro-atomes, pour autant qu'ils n'influent pas défavorablement sur le processus de synthèse.

On décrit ci-après, en référence à la nomenclature exposée au tableau I, successivement la préparation de lipopeptides hydrophobes et leur transformation en lipopeptides amphipatiques, et la préparation directe de lipopeptides amphitatiques.

A-1 Synthèse de lipopeptides hydrophobes ($CnEb_p$, $CnDb_p$ et $CnKt_p$)

On dissout l'amine grasse ($CnNH_2$) dans le chloroforme, puis on ajoute le NCA d'acide aminé approprié et on laisse polymériser à la température ambiante, sous agitation, pendant deux jours. On obtient ainsi les lipopeptides $CnEb_p$ possédant une séquence peptidique de poly(glutamate de benzyle), $CnDb_p$ possédant une séquence peptidique de poly(aspartate de benzyle), et $CnKt_p$ possédant une séquence peptidique de polytrifluoroacétyllysine.

A titre d'exemple, pour la synthèse de lipopeptides $C17Kt_p$, on utilise de l'heptadécylamine $C_{17}H_{35}NH_2$ obtenue à partir de l'acide stéarique (voir Indian J. Technol. *5*, 262 (1967). Pour obtenir plus précisément du $C17Kt_{10}$ de degré de polymérisation 10 (DP = 10), on ajoute à 2,55 g (0,01 mole) d'amine en C17 en solution dans 150 ml de chloroforme, 27 g (0,1 mole) de NCA de trifluoroacétyllysine et on laisse polymériser à température ambiante sous agitation pendant plusieurs heures. Le chloroforme est ensuite évaporé, le résidu est repris dans le méthanol et le polymère est précipité dans l'eau, puis est filtré, lavé et séché.

A-2 Transformation de lipopeptides hydrophobes en lipopeptides amphipatiques

1. Lipopeptides $CnK_p$

On a préparé les lipopeptides $CnK_p$ possédant une séquence hydrophile de polylysine (K) à partir des lipopeptides $CnKt_p$ par la méthode de Sela et al. [Biopolymers, *1*, 517 (1963)]. On traite les lipopeptides $CnKt_p$ en solution dans le THF, d'abord par une solution de pipéridine dans le méthanol, puis par une solution de pipéridine dans l'eau.

A titre d'exemple illustrant une transformation de $C17Kt_p$ en $C17K_p$, 5 g de $C17Kt_p$ sont dissous dans 150 ml d'une solution molaire de pipéridine dans le méthanol à température ambiante. Après 2 heures, on ajoute 100 ml d'une solution molaire de pipéridine dans l'eau et on laisse 48 heures à température ambiante. On élimine le méthanol à l'évaporateur et on passe la solution aqueuse sur une colonne de résine échangeuse d'anions (Duolite A 102 D, forme $OH^-$) pour en éliminer les anions trifluoroacétate et on lyophilise l'éluat pour récupérer le $C17K_p$.

2. Lipopeptides $CnE_p$ et $CnD_p$

On obtient les lipopeptides $CnE_p$ possédant une séquence hydrophile d'acide poly-glutamique (E) et $CnD_p$ possédant une séquence hydrophile d'acide poly-aspartique (D) à partir des lipopeptides $CnEb_p$ et $CnDb_p$ en traitant ces lipopeptides par HCl et HBr à température ambiante [J. Am. Chem. Soc., *80* ; 4631 (1958)].

3. Lipopeptides $CnEp_p$

On prépare les lipopeptides $CnEp_p$ possédant une séquence hydrophile de poly-hydroxypropylglutamine (Ep) en traitant les lipopeptides $CnEb_p$ par l'aminopropanol à 60 °C en solution dans le dioxane [Biopolymers, *3*, 625 (1965)].

4. Lipopeptides $CnEe_p$

On prépare les lipopeptides $CnEe_p$ possédant une séquence hydrophile de poly-hydroxyéthylglutamine (Ee) en traitant les lipopeptides $CnEb_p$ par l'éthanolamine à 60 °C en solution dans le dioxane [Biopolymers, *9*, 717 (1970)].

B — Synthèse directe de lipopeptides amphipatiques

On dissout l'amine grasse $CnNH_2$ dans le chloroforme, puis on ajoute le NCA d'acide aminé et on laisse polymériser, à température ambiante, sous agitation, pendant deux jours. On obtient ainsi les lipopeptides $CnSar_p$ possédant une séquence peptidique de polysarcosine.

Pour illustrer plus concrètement le procédé de synthèse de lipopeptides selon l'invention, on décrit ci-après la synthèse des lipopeptides amphipatiques $C17Sar_p$ formés d'une chaîne aliphatique contenant 17 atomes de carbone (C17) et d'une chaîne de polysarcosine $(Sar)_p$ et celle de $C12Sar_{20}$ et de $C18Sar_{11}$.

1. Synthèse de lipopeptides de degré de polymérisation supérieur à 3

a) Synthèse de $C17Sar_{20}$

L'heptadécylamine ($C17\,NH_2$) obtenue à partir de l'acide stéarique [voir Indian J. Technol., *5*, 262 (1967)] est d'abord dissoute dans le chloroforme, puis on y ajoute la quantité de NCA de sarcosine calculée pour obtenir le degré de polymérisation choisi. Par exemple, si on veut obtenir du $C17Sar_{10}$ de degré de polymérisation 10 (DP : 10), à 2,55 g (0,01 mole) d'amine $C_{17}H_{35}NH_2$ en solution dans 100 ml de chloroforme, on ajoute 11,5 g de NCA de sarcosine (0,1 mole) et on laisse polymériser à température ambiante, sous agitation pendant 48 heures.

On fractionne les lipopeptides $C17Sar_p$ par précipitation fractionnée en utilisant le diméthylformamide comme solvant et l'acétone comme précipitant.

b) Synthèse de $C12Sar_{20}$

On ajoute 23 g (0,2 mole) de NCA de sarcosine à 1,85 g (0,01 mole) de dodécylamine en solution dans 100 ml de chloroforme et on laisse polymériser à température ambiante, sous agitation, pendant 48 heures. On peut fractionner les lipopeptides $C12Sar_p$ par précipitation fractionnée en utilisant les solvants précipitants diméthylformamide/acétone.

On obtient xg du produit cherché solide blanc dont on a déterminé le degré de polymérisation par dosage de la fonction amine terminale après avoir vérifié le degré de pureté par chromatographie.

c) Synthèse de $C18Sar_{11}$

préparé en appliquant les mêmes conditions opératoires que selon b) avec 0,11 mole de NCA de sarcosine (12,65 g) et 0,01 mole de $C18NH_2$ (2,69 g), dans 100 ml de chloroforme on obtient le $C18Sar_{11}$ avec un rendement supérieur à 80 %, dont le spectre infrarouge dans le KBr est l'objet de la figure 1.

2. Synthèse de lipopeptides de degrés de polymérisation 1, 2 et 3

Pour obtenir des lipopeptides de degrés de polymérisation 1, 2 et 3, on peut procéder par couplage peptidique entre l'amine grasse et l'amino acide N-protégé par le groupement tert-butyl-oxycarbonyle (Boc).

Les Boc-amino acides sont préparés à partir de l'amino acide et du di-tert-butyl-dicarbonate par la méthode de Morsder et al [Hoppe-Seyler's Z. Physiol. Chem., *357*, 1651 (1976)].

a) Synthèse de $C17Sar_1$

α) $C17BocSar_1$ : on obtient le produit $C17BocSar_1$ en couplant l'heptadécylamine au $BocSar_1$ en présence de dicyclohexylcarbodiimide (DCCI). On mélange à froid (à 0 °C), dans 100 ml de chloroforme, 3,78 g (0,02 mole) de BocSar et 2,06 g (0,01 mole) de DCCI. Il se forme un précipité abondant de dicyclohexylurée (DCU). On laisse 30 minutes à 0 °C sous agitation et on ajoute 2,55 g (0,01 mole) d'heptadécylamine. On laisse la réaction se poursuivre pendant 20 heures. On filtre, on lave le précipité, on récupère le filtrat, on évapore le chloroforme, on reprend le résidu dans 50 ml de THF, on refroidit à 0 °C, on filtre pour éliminer le maximum de dicyclohexylurée, et on lave le précipité avec un minimum de THF froid.

β) $C17Sar_1$, HCl : au filtrat, on ajoute 20 ml d'HCl en solution 5N dans du THF et on laisse sous agitation pendant 24 heures à température ambiante. Il se forme un précipité abondant de $C17Sar_1$, HCl, qu'on filtre et lave au THF.

γ) $C17Sar_1$ : on reprend le précipité dans 100 ml de THF et on chauffe à 50 °C, puis on ajoute 2 ml de

triéthylamine et on laisse reposer pendant 2 heures. On refroidit à 0 °C, on filtre le précipité de chlorhydrate de triéthylamine, on évapore le THF et on recristallise le C17 $Sar_1$ dans l'acétone.

On obtient 2,1 g de $C17Sar_1$ (rendement 65 %). F = 58 °C

b) Synthèse de $C17Sar_2$

Pour obtenir du $C17Sar_2$, on opère de la même manière, mais on part de $C17Sar_1$ et de BocSar. F = 74 °C

c) Synthèse de $C17Sar_3$

Pour obtenir du $C17Sar_3$, on opère de la même manière, mais on part de $C17Sar_2$ et de BocSar. F = 83 °C

d) Synthèse de $C12Sar_1$

préparation de C12SarBoc

On dissout dans 100 ml de chloroforme, 1,85 g (0,01 mole) de dodécylamine, 1,89 g (0,01 mole) de SarBoc et 1,15 g (0,01 mole) de N-hydroxysuccinimide puis on ajoute, sous agitation, 2,06 g de dicyclohexylcarbodiimide et maintient l'agitation pendant 24 heures. On élimine alors le précipité de dicyclohexylurée, évapore le solvant du filtrat et reprend le résidu dans 100 ml d'acétone pour éliminer le reste de dicyclohexylurée solide. Le produit recherché précipite lorsqu'on ajoute au filtrat un volume d'eau. Après lavage du précipité par un mélange acétone/eau, on obtient xg de C12SarBoc.

préparation du chlorhydrate de C12Sar

Le produit précédemment obtenu est dissous dans 80 ml de THF ; on ajoute 20 ml d'acide chlorhydrique 5N en solution dans l'éther diéthylique et abandonne 24 heures à température ambiante pendant lesquelles le chlorhydrate final précipite. On l'isole par filtration à 0 °C, le lave au THF glacé et le sèche sous vide. On obtient ainsi le C12Sar, HCl.

isolement de $C12Sar_1$

Le sel précédemment obtenu est dissous dans 50 ml de méthanol ; on ajoute 100 ml de solution aqueuse 0,1N de soude et on évapore les solvants sous vide à température ambiante jusqu'à 25 ml environ. On verse cette solution dans 100 ml d'eau et extrait la phase aqueuse par 100 ml d'acétate d'éthyle puis par 50 ml du même solvant. Les phases organiques sont réunies et séchées sur sulfate de sodium. Le solvant est alors éliminé sous vide à 0 °C.

Le résidu est purifié par chromatographie sur une colonne de gel de silice, avec comme éluant une solution de méthanol contenant 1 % en volume de solution aqueuse d'ammoniaque (à 30 % environ).

On obtient ainsi 2,05 g de $C12Sar_1$ de point de fusion 39 °C.

e) Synthèse de $C12Sar_2$ et $C12Sar_3$

On applique le même procédé que celui décrit pour la synthèse de $C12Sar_1$ mais en utilisant comme matière première respectivement le $C12Sar_1$ et le $C12Sar_2$.

Les rendements sont comparables.

$C12Sar_2$ : point de fusion 58 °C.
$C12Sar_3$ : point de fusion 66-67 °C.

f) Synthèse des $C16Sar_{1,2,3}$

On applique les modes opératoires décrits selon d) et e) pour préparer ces dérivés dont les spectres infra-rouges (en KBr) sont représentés respectivement aux figures 2, 3 et 4.

g) Synthèse de $C18Sar_2$

On ajoute par petites portions à une solution de 2,69 g (0,01 mole) d'octadécylamine dans 150 ml de chloroforme, 2,3 g (0,02 mole) de NCA de sarcosine, sous bonne agitation. On élimine alors le solvant, on dissout le résidu dans l'éther diéthylique puis l'élimine sous vide à température voisine de 0 °C.

On obtient 4 g de produit dont le degré de polymérisation moyen, déterminé par dosage de la fonction amine terminale par l'acide perchlorique dans l'acide acétique est très voisin de 2.

Par chromatographie sur colonne de gel de silice en éluant avec du méthanol contenant 1 % de

solution aqueuse concentrée d'ammoniaque, on sépare du C18$Sar_1$, C18$Sar_2$, C18$Sar_3$, C18$Sar_4$ pour obtenir plus de 60 % de C18$Sar_2$. Il y a aussi un peu de C18$Sar_5$ et une trace de C18$Sar_6$ avec un reste d'amine de départ.

Le C18$Sar_2$ ainsi obtenu, même non séparé de ses homologues, a des propriétés émulsifiantes comparables à celles du C18$Sar_2$ obtenu par la méthode au SarBoc.

La structure des lipopeptides selon l'invention a été étudiée par la technique de diffraction aux rayons X.

On a ainsi pu établir que les lipopeptides amphipatiques présentent des mésophases à structure périodique en solution aqueuse pour des concentrations en eau inférieures à 60 % environ et que la structure périodique peut être conservée à l'état sec par évaporation lente de l'eau de la mésophase. Les lipopeptides amphipatiques selon l'invention constituent ainsi une nouvelle classe de cristaux liquides liotropes et ils peuvent avoir les mêmes applications que ces cristaux liquides.

La structure des lipopeptides amphipatiques est maintenant décrite plus en détails en référence à l'exemple des lipopeptides C17$Sar_p$ constitués d'une chaîne aliphatique possédant 17 atomes de carbone et d'une chaîne de polysarcosine, dont on a fait varier le degré de polymérisation de 1 à 60.

Les lipopeptides C17$Sar_p$ présentent un double polymorphisme : en fonction de la longueur de la chaîne polypeptidique d'une part et en fonction de la teneur en eau d'autre part. Les lipopeptides adoptent, suivant leur composition, trois types de structures : lamellaires pour les degrés de polymérisation (DP) inférieurs à 9, hexagonale pour les DP compris entre 10 et 35 environ et cubique centrée pour les DP supérieurs à environ 35. De plus, les lipopeptides peuvent présenter un polymorphisme en fonction de la teneur en eau de leurs mésophases. L'addition d'eau modifie le rapport entre les volumes des phases hydrophile et hydrophobe et peut faire passer la structure de lamellaire à hexagonale (pour les DP compris entre 5 et 9 environ) ou d'hexagonale à cubique (pour les DP compris entre 17 et 35 environ).

L'invention a donc également pour objet l'application des lipopeptides amphipatiques à la constitution de cristaux liquides liotropes.

Les mésophases de lipopeptides amphipatiques peuvent en outre incorporer de nombreux composants, tant hydrophiles qu'hydrophobes, tels que : alcools, acides, paraffines, huile de carnation, stéarate d'éthyle, palmitate d'isopropyle, etc., et donner ainsi par exemple des laits ou des crèmes, dont on peut faire varier facilement la viscosité en jouant sur la structure des mésophases, elle-même déterminée par la longueur respective des séquences hydrophobes et peptidiques des lipopeptides.

On a également testé les propriétés émulsifiantes des lipopeptides amphipatiques vis-à-vis de nombreux couples de liquides non miscibles tels que eau/hydrocarbures et eau/produits de base de l'industrie des cosmétiques. On a étudié le type et la stabilité des émulsions obtenues par la méthode des colorants sélectifs, la méthode des dilutions, la conductivité électrique, la cryofracture et la microscopie électronique.

Pour obtenir des émulsions, on ajoute aux deux liquides non miscibles environ 1 % en poids de lipopeptide amphipatique (Cn$Sar_p$ avec n = 16, 17 ou 18 et p = 1, 2 ou 3 par exemple), on agite 10 à 15 minutes et l'émulsion se forme facilement. On a ainsi préparé des émulsions de différentes compositions, de 30 à 70 % de chaque composant, avec les systèmes eau/octane, eau/myristate d'isopropyle, eau/palmitate d'isopropyle, eau/stéarate de butyle ou d'éthyle, eau/huile de carnation, eau/huile de vaseline, eau/cosbiol, eau/mygliol). Les émulsions obtenues sont très stables (plusieurs mois) et résistent à des élévations de température jusqu'à 60 °C environ. On modifie la viscosité, la compacité et l'onctuosité des émulsions en faisant varier la teneur en lipopeptides entre 1 et 2 %.

L'invention a donc en outre pour objet l'application des lipopeptides amphipatiques comme émulsifiants et les émulsions comprenant des lipopeptides amphipatiques à titre d'agent émulsifiant, présent en une quantité de l'ordre de 1 % en poids ou plus.

On peut faire varier à volonté la solubilité et l'affinité pour différents solvants des lipopeptides en modifiant le nombre d'atomes de carbone de la chaîne hydrophobe et la nature de la chaîne peptidique. On peut aussi modifier facilement la balance hydrophile-hydrophobe de tels lipopeptides en modifiant le nombre d'atomes de carbone de la chaîne hydrophobe et le degré de polymérisation de la chaîne peptidique.

Les lipopeptides amphipatiques selon l'invention donnent facilement des émulsions très stables pour des teneurs en lipopeptides très faibles (environ 1 % en poids) alors qu'il est nécessaire d'avoir 15-16 % des agents de surface classiques. De plus, ils ont l'avantage d'être réalisés avec des composants naturels (lipides et peptides). Ces lipopeptides amphipatiques peuvent trouver une application comme agents émulsifiants de milieux non miscibles dans des domaines très divers, par exemple dans l'industrie cosmétique (crèmes hydratantes, crèmes antirides, vernis, dissolvants de vernis, etc.), dans l'industrie alimentaire (moutardes, mayonnaises, etc.) et dans l'industrie pétrolière (additifs pour huiles, récupération assistée de pétrole), entre autres.

(Voir tableau I page 7)

Tableau I

Nomenclature des séquences peptidiques

| Désignation | Nom du polypeptide | Formule de la chaîne latérale |
|---|---|---|
| Eb | Poly(glutamate de benzyle) | $-(CH_2)_2-COO-CH_2-C_6H_5$ |
| Ep | Poly(hydroxypropylglutamine) | $-(CH_2)_2-CO-NH-(CH_2)_3OH$ |
| Ee | Poly(hydroxyéthylglutamine) | $-(CH_2)_2-CO-NH-(CH_2)_2OH$ |
| E | Poly(acide glutamique) | $-(CH_2)_2-COOH$ |
| Db | Poly(aspartate de benzyle) | $-CH_2-COO-CH_2-C_6H_5$ |
| D | Poly(acide aspartique) | $-CH_2-COOH$ |
| Kt | Poly(trifluoroacétyllysine) | $-(CH_2)_4-NH-CO-CF_3$ |
| K | Polylysine | $-(CH_2)_4-NH_2$ |
| S | Polysérine | $-CH_2-OH$ |
| T | Polythréonine | 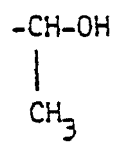 |
| Sar | Polysarcosine (+) | |

(+) Polysarcosine :

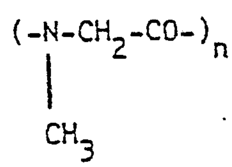

**Revendications**

1. Lipopeptides, caractérisés en ce qu'ils sont amphipatiques et sont constitués d'une chaîne hydrophobe comprenant de 8 à 24 atomes de carbone environ terminée par un groupement amino engagé dans une liaison amide avec le groupe carboxyle terminal d'une chaîne polypeptidique hydrophile ou rendue hydrophile.

2. Lipopeptides selon la revendication 1, caractérisés en ce qu'ils répondent à la formule générale :

CnPP

dans laquelle Cn représente une chaîne hydrophobe ayant de 8 à 24 atomes de carbone environ, et PP représente un polypeptide obtenu à partir des acides aminés naturels ou de leurs dérivés.

3. Lipopeptides selon les revendications 1 ou 2, caractérisés en ce que le polypeptide est obtenu à partir de la sarcosine.

4. Lipopeptides selon les revendications 1 ou 2, caractérisés en ce que le polypeptide est obtenu à partir de la lysine.

5. Lipopeptides selon les revendications 1 ou 2, caractérisés en ce que le polypeptide est obtenu à partir de l'acide glutamique.

6. Lipopeptides selon les revendications 1 à 3, caractérisés en ce qu'ils répondent à la formule $CnSar_{1\text{-}3}$ avec n choisi parmi 16, 17 et 18.

7. Procédé pour l'obtention de lipopeptides selon l'une des revendications 1 à 6, caractérisé en ce qu'il consiste fondamentalement à :

1) réaliser un couplage peptidique entre une amine grasse et un amino acide N-protégé, pour obtenir un lipopeptide dont la chaîne peptidique a un degré de polymérisation de 1 et, si l'on désire obtenir un degré de polymérisation de 2 ou 3 pour la chaîne peptidique, réaliser un autre couplage peptidique d'un amino acide N-protégé sur le produit de degré de polymérisation immédiatement inférieur, ou

2a) effectuer la polymérisation du N-carboxyanhydride de l'amino acide en l'initiant par l'amine grasse $CnNH_2$, et

2b) si on le désire, fractionner en composition les lipopeptides de l'étape 2a), et

3) excepté dans le cas où la chaîne peptidique du produit de l'étape 1) et/ou 2a) ou 2b) est directement une chaîne hydrophile, débloquer les chaînes latérales de la chaîne peptidique hydrophobe pour les rendre hydrophiles.

8. Procédé selon la revendication 7 pour l'obtention de lipopeptides ayant un degré de polymérisation de 1, 2 ou 3, caractérisé en ce qu'on effectue un couplage peptidique entre une amine grasse de formule $CnNH_2$ ou Cn est tel que précédemment défini, et l'amino acide dont l'atome d'azote amino est protégé, par exemple par le groupement tert-butyloxycarbonyle, obtenant ainsi un lipopeptide ayant un degré de polymérisation de 1, et pour obtenir des lipopeptides ayant un degré de polymérisation de 2 ou 3 pour la chaîne peptidique, on réalise un autre couplage peptidique d'un amino acide N-protégé sur le produit de degré de polymérisation immédiatement inférieur.

9. Procédé selon la revendication 7 pour l'obtention de lipopeptides, caractérisé en ce qu'on polymérise le N-carboxy-anhydride de l'amino acide en initiant la polymérisation par une amine grasse $CnNH_2$ où Cn est tel que précédemment défini, et on réalise ensuite, si on le désire, un fractionnement en composition par précipitation sélective des lipopeptides, obtenant ainsi une série de lipopeptides qui diffèrent par leur composition en peptides.

10. Application des lipopeptides selon l'une des revendications 1 à 6 à l'obtention de cristaux liquides liotropes.

11. Cristaux liquides, caractérisés en ce qu'ils comprennent au moins un lipopeptide selon l'une des revendications 1 à 6.

12. Application des lipopeptides selon l'une des revendications 1 à 6 comme émulsifiants.

13. Emulsions de milieux non miscibles, caractérisées en ce qu'elles comprennent, au moins 0,8 % environ d'un lipopeptide selon l'une des revendications 1 à 6.

**Claims**

1. Lipopeptides, characterized in that they are amphiphatic and consist of a hydrophobic chain comprising from 8 to 24 carbon atoms approximately and ending in an amino group involved in an amide bond with the carboxyl-terminal group of a polypeptide chain which is hydrophilic or is made hydrophilic.

2. Lipopeptides according to Claim 1, characterized in that they correspond to the general formula :

$$CnPP$$

in which Cn denotes a hydrophobic chain having from 8 to 24 carbon atoms approximately, and PP denotes a polypeptide obtained from natural amino acids or from derivatives thereof.

3. Lipopeptides according to Claim 1 or 2, characterized in that the polypeptide is obtained from sarcosine.

4. Lipopeptides according to Claim 1 or 2, characterized in that the polypeptide is obtained from lysine.

5. Lipopeptides according to Claim 1 or 2, characterized in that the polypeptide is obtained from glutamic acid.

6. Lipopeptides according to Claims 1 to 3, characterized in that they correspond to the formula $CnSar_{1\text{-}3}$ where n is chosen from 16, 17 and 18.

7. Process for obtaining lipopeptides according to one of Claims 1 to 6, characterized in that it consists essentially in :

1) carrying out a peptide coupling between a fatty amine and an N-protected amino acid to obtain a lipopeptide in which the peptide chain has a degree of polymerization of 1 and, if it is desired to obtain a

degree of polymerization of 2 or 3 for the peptide chain, carrying out a further peptide coupling of an N-protected amino acid with the product having a degree of polymerization lying immediately below, or

2a) performing polymerization of the N-carboxy-amino acid anhydride, initiating it with the fatty amine $CnNH_2$, and

2b) if so desired, fractionating in terms of their composition the lipopeptides of stage 2a), and

3) except in the case where the peptide chain of the product of stage 1) and/or 2a) or 2b) is directly a hydrophilic chain, unblocking the side-chains of the hydrophobic peptide chain to make them hydrophilic.

8. Process according to Claim 7 for obtaining lipopeptides having a degree of polymerization of 1, 2 or 3, characterized in that peptide coupling is performed between a fatty amine of formula $CnNH_2$, where Cn is as defined above, and the amino acid in which the amino nitrogen atom is protected, for example by a tert-butyloxycarbonyl group, a lipopeptide having a degree of polymerization of 1 thereby being obtained, and, to obtain lipopeptides having a degree of polymerization of 2 or 3 for the peptide chain, a further peptide coupling of an N-protected amino acid is carried out with the product having a degree of polymerization lying immediately below.

9. Process according to Claim 7 for obtaining lipopeptides, characterized in that the N-carboxy amino acid anhydride is polymerized, the polymerization being initiated with a fatty amine $CnNH_2$, where Cn is as defined above and, if so desired, a fractionation of the lipopeptides in terms of their composition is then carried out by selective precipitation, a series of lipids which differ in their peptide composition thereby being obtained.

10. Application of the lipopeptides according to one of Claims 1 to 6 to the production of lipotropic liquid crystals.

11. Liquid crystals, characterized in that they contain at least one lipopeptide according to one of Claims 1 to 6.

12. Application of the lipopeptides according to one of Claims 1 to 6 as emulsifiers.

13. Emulsions of immiscible media, characterized in that they contain at least approximately 0.8 % of a lipopeptide according to one of Claims 1 to 6.

**Patentansprüche**

1. Lipopeptide, dadurch gekennzeichnet, daß sie amphipatisch sind und aus einer hydrophoben Kette, enthaltend ungefähr 8 bis 24 C-Atome besteht, mit einer endständigen Aminogruppierung, welche mittels einer Amidbindung mit der endständigen Carboxylgruppe einer hydrophilen oder hydrophil gemachten Polypeptidkette verbunden ist.

2. Lipopeptide gemäß Anspruch 1, dadurch gekennzeichnet, daß sie der allgemeinen Formel

CnPP

entsprechen, in der Cn eine hydrophobe Kette mit ungefähr 8 bis 24 C-Atomen darstellt und PP ein Polypeptid, erhalten aus natürlichen Aminosäuren oder ihren Derivaten, darstellt.

3. Lipopeptide gemäß den Ansprüchen 1 oder 2, dadurch gekennzeichnet, daß das Polypeptid aus Sarkosin erhalten ist.

4. Lipopeptide gemäß den Ansprüchen 1 oder 2, dadurch gekennzeichnet, daß das Polypeptid aus Lysin erhalten worden ist.

5. Lipopeptide gemäß den Ansprüchen 1 oder 2, dadurch gekennzeichnet, daß das Polypeptid aus Glutaminsäure erhalten worden ist.

6. Lipopeptide gemäß den Ansprüchen 1 bis 3, dadurch gekennzeichnet, daß sie der Formel $CnSar_{1-3}$ mit n ausgewählt aus 16, 17 und 18 entsprechen.

7. Verfahren zur Herstellung von Lipopeptiden gemäß einem der Ansprüche 1 bis 6, dadurch gekennzeichnet, daß es im wesentlichen besteht aus :

1) Durchführung einer Peptidkopplung zwischen einem Fettamin und einer N-geschützten Aminosäure, um ein Lipopeptid zu erhalten, dessen Peptidkette einen Polymerisationsgrad von 1 aufweist, und, wenn man es wünscht, einen Polymerisationsgrad von 2 oder 3 für die Peptidkette zu erhalten, dem Durchführen einer weiteren Peptidkopplung einer N-geschützten Aminosäure mit dem Produkt mit einem unmittelbar tiefer liegenden Polymerisationsgrad, oder

2a) der Durchführung der Polymerisation des N-Carboxy-anhydrids der Aminosäure, indem man sie mittels der Fettsäure $CnNH_2$ in Gang setzt, und

2b) wenn man es wünscht, die Lipopeptide der Stufe 2a) aus der Zusammensetzung zu fraktionieren, und

3) ausgenommen in dem Fall, in welchem die Peptidkette des Produktes der Stufe 1) und/oder 2a) oder 2b) unmittelbar eine hydrophile Kette ist, dem Deblockieren der Seitenkette der hydrophoben Peptidkette, um sie hydrophil zu machen.

8. Verfahren gemäß Anspruch 7 zur Herstellung von Lipopeptiden mit einem Polymerisationsgrad von 1, 2 oder 3, dadurch gekennzeichnet, daß man eine Peptidkopplung zwischen einem Fettamin der

Formel $CnNH_2$ in der Cn wie vorstehend definiert ist, und der Aminosäure, deren Aminostickstoffatom geschützt ist, z. B. mittels einer t-Butyloxycarbonylgruppierung, durchführt und so ein Lipopeptid mit einem Polymerisationsgrad von 1 erhält und, um Lipopeptide mit einem Polymerisationsgrad von 2 oder 3 für die Peptidkette zu erhalten, man eine weitere Peptidkopplung einer N-geschützten Aminosäure mit dem Produkt mit einem unmittelbar darunterliegenden Polymerisationsgrad durchführt.

9. Verfahren gemäß Anspruch 7 zur Herstellung von Lipopeptiden, dadurch gekennzeichnet, daß man das N-Carboxy-Anhydrid der Aminosäure polymerisiert, indem man die Polymerisation durch ein Fettamin $CnNH_2$, worin Cn wie oben definiert ist, in Gang setzt und anschließend, wenn man es wünscht, aus dem Gemisch eine Fraktionierung mittels selektiver Fällung der Lipopeptide bewirkt und so eine Reihe von Lipopeptiden erhält, die sich durch Ihre Peptidzusammensetzung unterscheiden.

10. Verwendung von Lipopeptiden gemäß einem der Ansprüche 1 bis 6 zur Herstellung von lipotropen Flüssigkristallen.

11. Flüssigkristalle, dadurch gekennzeichnet, daß sie wenigstens ein Lipopeptid gemäß einem der Ansprüche 1 bis 6 enthalten.

12. Verwendung von Lipopeptiden gemäß einem der Ansprüche 1 bis 6 als Emulgator.

13. Emulsionen eines nichtmischbaren Mittels, dadurch gekennzeichnet, daß sie wenigstens ungefähr 0,8 % eines Lipopeptids gemäß einem der Ansprüche 1 bis 6 enthalten.

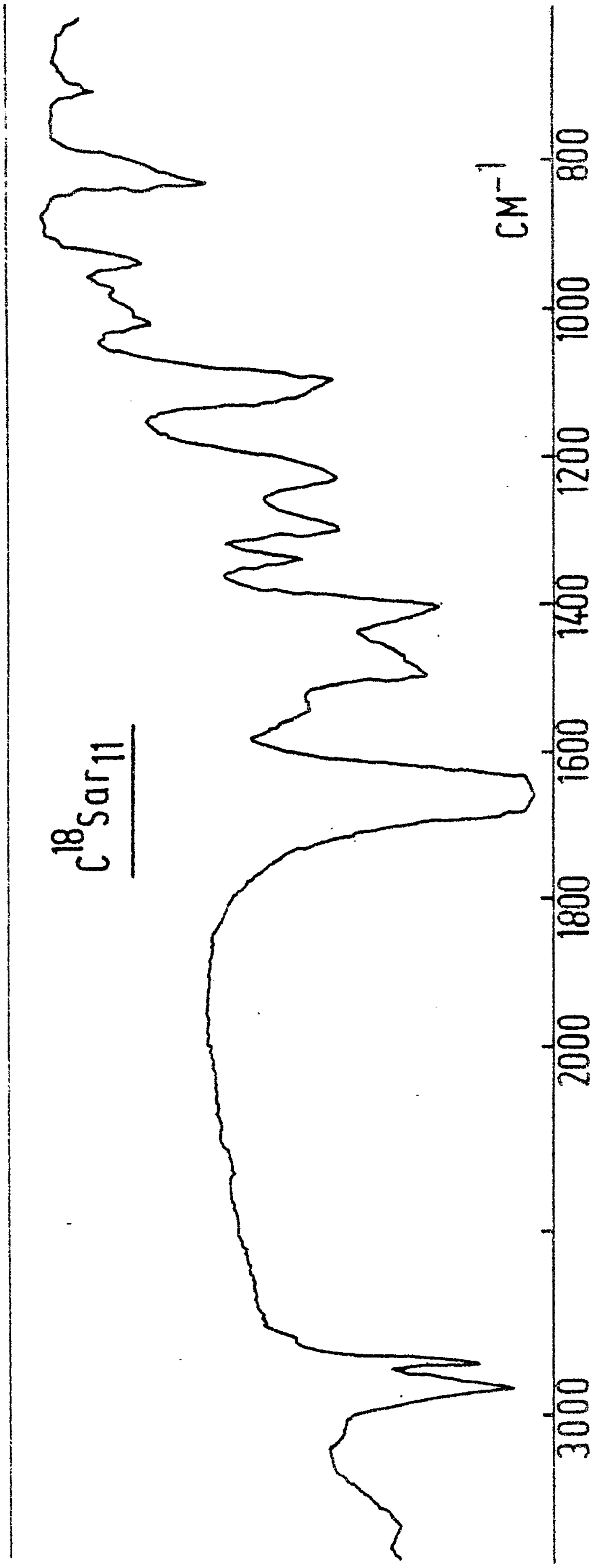


FIG.1

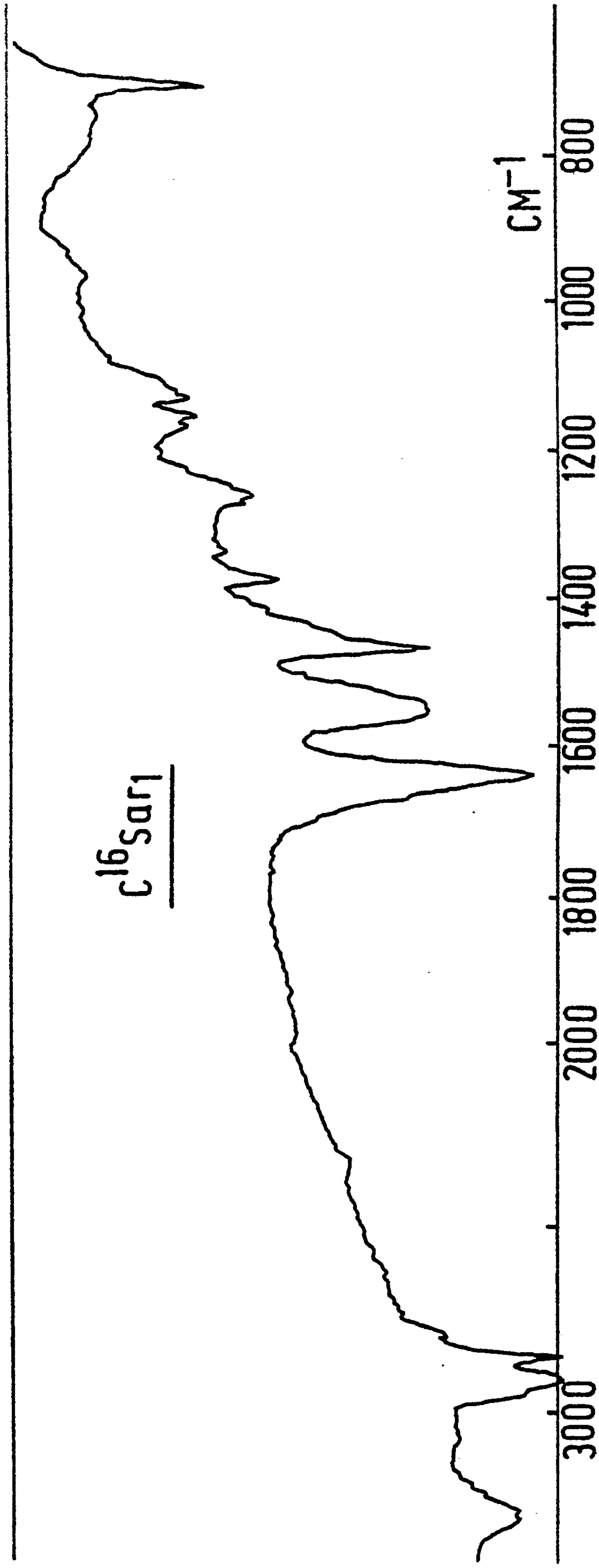


FIG. 2

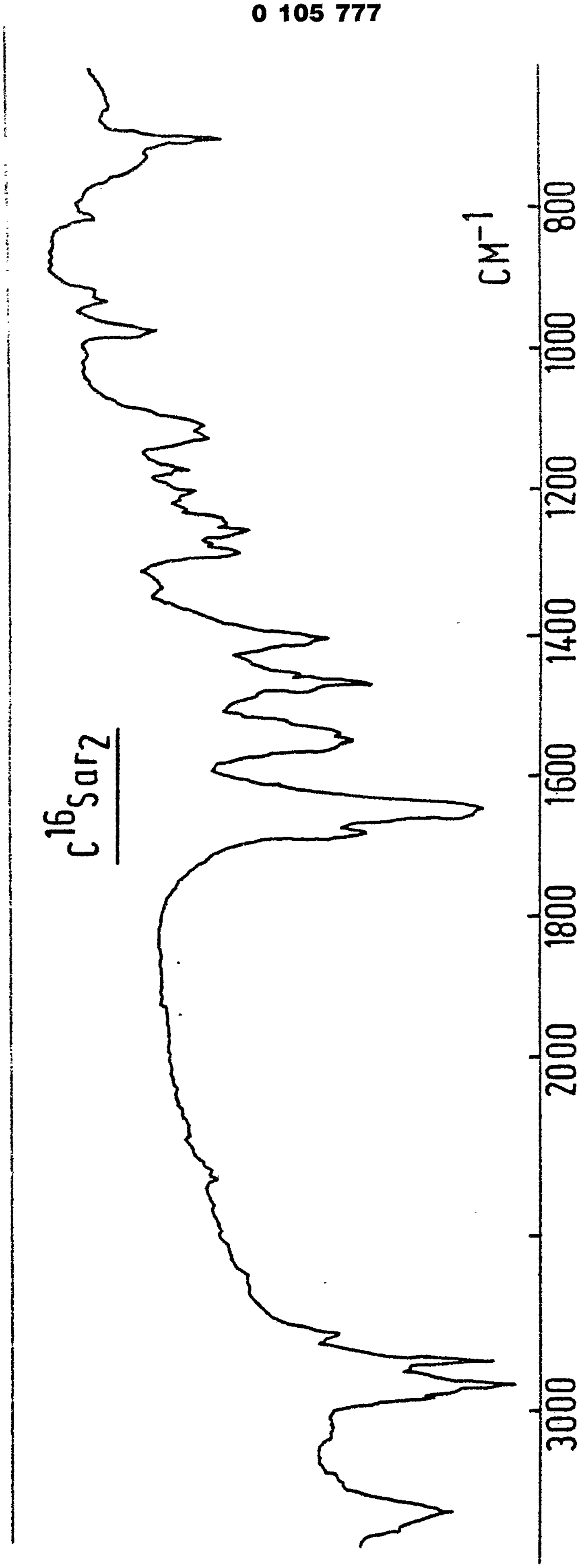


FIG. 3

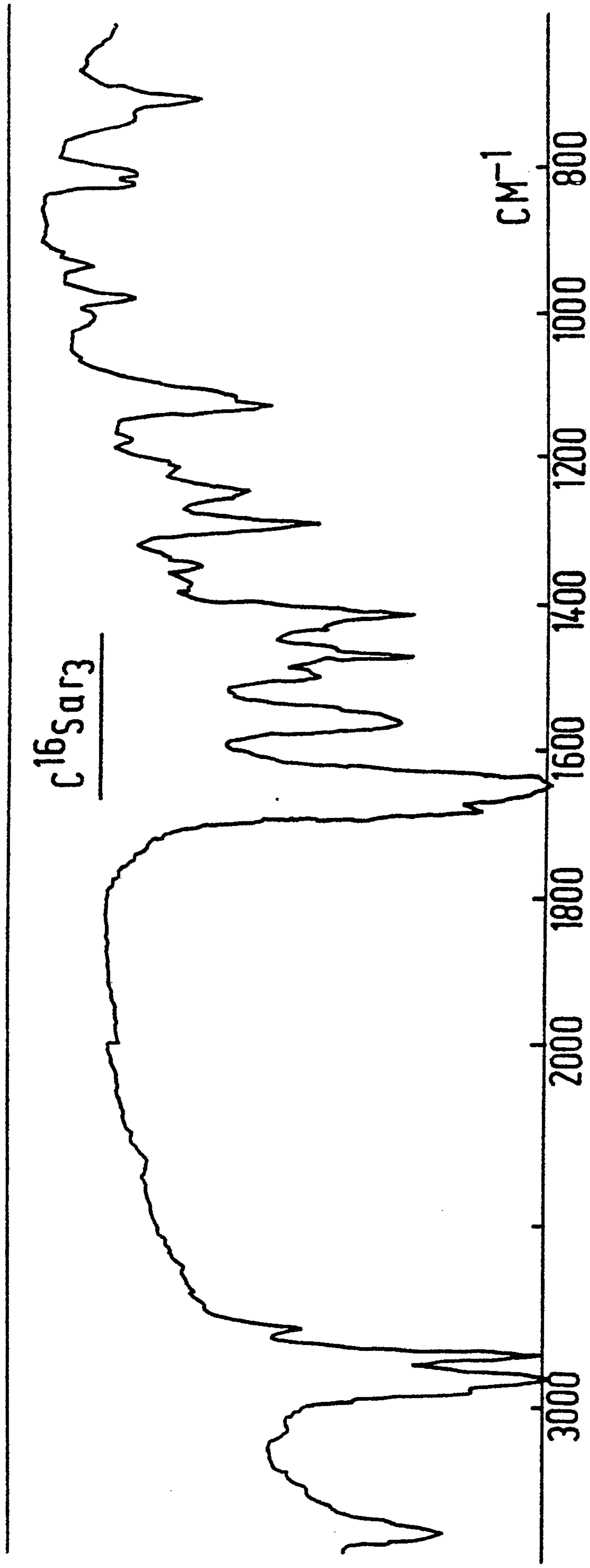


FIG. 4